# FASCICULE DE BREVET EUROPEEN

(11) **EP 0 930 908 B1**
(45) Date de publication et mention de la délivrance du brevet: **05.02.2003**
(21) Numéro de dépôt: 97940185.8
(22) Date de dépôt: 05.09.1997
(51) Int. Cl.: A61M 16/00, A61F 5/56, A61M 16/10

(54) **DISPOSITIF DESTINE A EVITER LE RONFLEMENT ET LES APNEES DU SOMMEIL**
VORRICHTUNG GEGEN SCHNARCHEN UND ATEMSTILLSTAND IM SCHLAF
DEVICE FOR PREVENTING SNORING AND APNOEA DURING SLEEP

(30) Priorité: 05.09.1996 FR 9610827
(43) Date de publication de la demande: 28.07.1999
(73) Titulaire: Aeroflux Medical International - AMI, 75016 Paris (FR)
(72) Inventeur: Aeroflux Medical International - AMI, 75016 Paris (FR)
(74) Mandataire: Colas, Jean-Pierre
(86) Numéro de dépôt international: FR9701569
(87) Numéro de publication internationale: WO98009675

(56) Documents cités:
- FR-A- 2 711 320
- GB-A- 299 457
- US-A- 3 057 347
- US-A- 4 222 378
- US-A- 5 402 776
- US-A- 5 529 062

## Description

La présente invention concerne un dispositif intra-bucco-pharyngé destiné au traitement de la ronchopathie.

Plus particulièrement, l'invention vise à supprimer le ronflement et à éviter les apnées du sommeil, survenant lors du sommeil de certains patients.

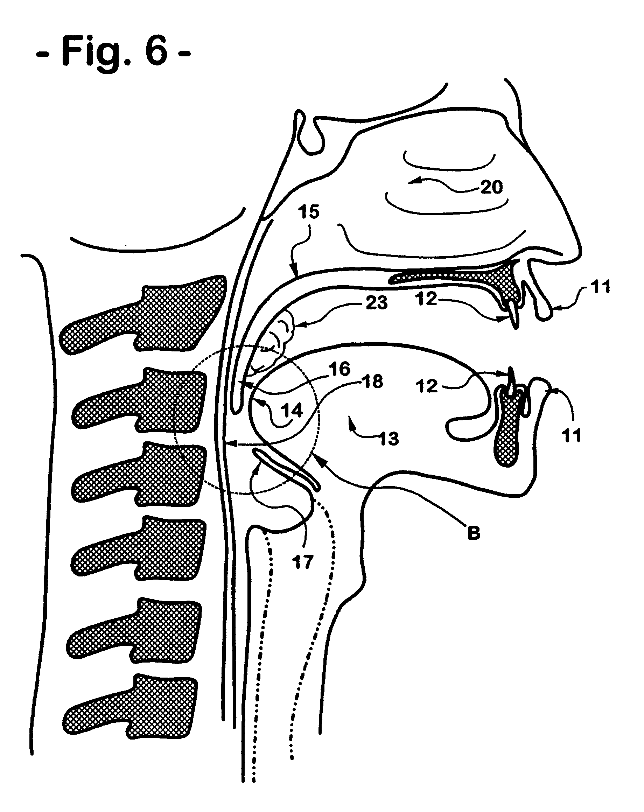

La figure 6 des dessins annexés permet un repérage anatomique et une meilleure compréhension des mécanismes du ronflement.

Le ronflement est un bruit inspiratoire, ou à prédominance inspiratoire, produit par la vibration des tissus mous pharyngés.

Le mécanisme du ronflement n'est pas encore totalement expliqué. Le ronflement résulterait de l'intrication de plusieurs anomalies: facteurs anatomiques locaux, parois pharyngées anormalement collabables, troubles du tonus musculaire et analogues.

De nombreuses anomalies peuvent réduire la lumière des voies aériennes supérieures : obstruction nasale par déviation de la cloison nasale 20, macroglossie ou hypertrophie amygdalienne 23, rétro ou micro-gnathisme.

Au cours du ronflement "bénin", le pharynx se rétrécit de façon importante. Cette réduction de la lumière pharyngée est le fait d'un voile du palais 15 et d'une luette 16 trop longs, descendant derrière la base 14 de la langue 13. L'oscillation de ces structures génère une vibration audible, le ronflement.

Chez les ronfleurs sévères, en plus du bruit, l'augmentation des résistances pharyngées limite le flux inspiratoire à différents niveaux, en particulier dans la région du voile du palais 15 et de la base de langue 14 qui sont plaqués contre la paroi pharyngée postérieure 18. Le décubitus dorsal favorise le ronflement, peut-être par chute de la langue 13, mais surtout de la base de langue 14 contre le mur pharyngé postérieur 18.

Les troubles occasionnés par le ronflement sont multiples. Outre la gêne pour l'entourage, le ronflement rend le sommeil de mauvaise qualité et diminue l'oxygénation sanguine de façon plus ou moins importante, ce qui peut avoir des conséquences néfastes sur tout l'organisme. Il semblerait que les ronfleurs sévères aient, plus souvent que les ronfleurs légers, une hypertension artérielle et des troubles ischémiques cardiaques à type d'angine de poitrine ou d'infarctus du myocarde. Ils auraient également une prédisposition à l'ischémie cérébrale.

Le syndrome d'apnées du sommeil (SAS) est une forme plus sévère du ronflement. Il s'agit en effet, d'un ronflement important, associé à des apnées réelles, plus ou moins longues, mais pouvant durer jusqu'à deux minutes et se répétant tout au long de la nuit, par obstruction totale de la filière aérienne pharyngée.

Ces arrêts respiratoires provoquent des réveils successifs (le système de sauvegarde fonctionnant) afin que les muscles pharyngés se contractent et laissent passer l'air inspiratoire.

Pendant la journée, le maître-symptôme est la somnolence. Elle est de sévérité variable, allant de la simple sensation de fatigue chronique jusqu'aux endormissements intempestifs en pleine activité.

Divers moyens ont été inventés et essayés afin de diminuer, voire supprimer, les ronflements: Oreillers spéciaux: (demande de brevet internationale WO-91/11157), "petits moyens" évitant de dormir sur le dos, médications diverses, systèmes électroniques réveillant partiellement le dormeur, dispositifs intra-buccaux d'écartement des maxillaires (demande de brevet EP0599445 et demande de brevet internationale WO-94/28832) ou de refoulement de la langue (demandes de brevet internationales WO-92/09249 ou WO-96/25193), mais tous, sans résultat probant.

Plus récemment, et du fait de la meilleure connaissance des mécanismes provoquant les bruits au cours du ronflement, la chirurgie du pharynx a été un progrès certain dans le traitement de ces troubles. En ce qui concerne le SAS, des respirateurs ont été mis au point afin d'insuffler un air sous pression positive afin d'éviter les apnées (C.P.A.P.: "Continuous Positive Air Pressure").

On constate que l'ensemble des méthodes et dispositifs mis en oeuvre dans la technique antérieure sont soit inefficaces, soit efficaces mais coûteux et/ou encombrants et pour le moins difficiles à utiliser par le patient lui-même hors du milieu hospitalier.

Par le brevet US 3 057 347, on connaît un dispositif de sauvetage respiratoire, destiné à la réanimation de patients souffrant d'arrêts respiratoires comme il en survient après une électrocution ou un noyade et comportant les caractéristiques énumérées dans le préambule de la revendication 1.

Plus précisément, ce dispositif comprend une canule présentant une partie proximale courbe et une partie distale droite, destinées à être insérées respectivement jusqu'à la base de la langue et à traverser la cavité buccale, tout en faisant saillie hors de celle-ci où un sauveteur peut insuffler de l'air. La partie distale est pourvue d'un disque d'appui qui l'entoure radialement et qui vient appuyer sur les lèvres du patient afin d'assurer un positionnement correct de l'ensemble. Un filtre placé dans la partie distale de la canule permet d'éviter que de l'humidité insufflée avec l'air respiratoire du sauveteur n'atteigne les voies respiratoires du patient. La canule est réalisée en un matériau semi-rigide, élastique afin de pouvoir s'adapter à la conformation anatomique particulière de chaque patient. L'application de ce dispositif est exclusivement réservée à la réanimation de patients et vise en particulier de créer un canal non obstrué entre la bouche du sauveteur et les voies respiratoires situées en aval de la base de la langue comme le larynx et la trachée. On observera que le patient étant inconscient, la canule doit présenter une rigidité telle qu'elle puisse être mise en place éventuellement en force, car les muscles pharyngés peuvent être tétanisés du fait du traumatisme.

Le dispositif de la technique antérieure présente l'inconvénient dans l'hypothèse où il serait utilisé pour traiter la ronchopathie ou les apnées du sommeil, que le tube risque de s'écraser au cours de l'utilisation, car contrairement à un patient en réanimation, un patient souffrant de ronchopathie, conserve, pendant le sommeil un certain tonus musculaire qui tend à écraser le tube par serrage des lèvres et des dents. Or, le dispositif antérieur comporte un moyen de renfort qui ne peut être utile que pour le réanimateur, mais non pas pour le patient.

La présente invention a pour but de proposer un dispositif simple et efficace de traitement de la ronchopathie et des apnées du sommeil, qui évite tous les inconvénients des dispositifs et méthodes, chirurgicales ou autres, utilisés jusqu'ici, le dispositif étant particulièrement commode à utiliser et pouvant être facilement mis en place par un patient souffrant de la ronchopathie et/ou des apnées du sommeil.

L'invention a donc pour objet un dispositif de traitement de la ronchopathie et des apnées du sommeil, présentant les caractéristiques définies dans la partie caractérisante de la revendication 1.

Des particularités avantageuses du dispositif de traitement selon l'invention sont définies dans les sous-revendications 2 à 12.

D'autres caractéristiques et avantages de l'invention apparaîtront au cours de la description qui va suivre donnée uniquement à titre d'exemple et faite en se référant aux dessins annexés sur lesquels:
- la figure 1 représente de profil, le dispositif selon l'invention;
- la figure 2 représente de face, le dispositif selon l'invention vu du côté proximale;
- la figure 3 représente de profil, et en coupe, le dispositif selon l'invention;
- la figure 4 représente de face, et en coupe, le dispositif selon l'invention;
- la figure 5 représente une coupe transversale du tube du dispositif selon l'invention.
- la figure 6 représente une coupe anatomique de l'oro-pharynx;
- la figure 7 représente le dispositif en place dans l'oro-pharynx et au niveau de la région pré-buccale, et
- la figure 7A est une vue partielle du dispositif au niveau de la région pré-buccale pour montrer une variante de l'invention.

En référence à ces dessins, le dispositif comporte selon une première caractéristique, un tube 1 intra-bucco-pharyngé souple et non rigide, afin d'être bien supportée par le patient lors de la mise en place avant le sommeil et pendant le sommeil. Ce tube 1 est de forme anatomique adaptée, c'est à dire arciforme pour se placer entre la langue 13 et le voile du palais 15, ce qui permet le passage de l'air.

Le matériau souple, non rigide du tube doit être compatible avec le contact de la muqueuse buccale. Il faut que la souplesse soit adaptée afin que les parois entre lesquelles il est inséré ne se collabent pas sous la pression des tissus du voile du palais et de la langue.

Il est important que le tube soit déformable dans une certaine mesure, mais qu'il reprenne une forme en arc lorsque l'effort de déformation est relâchée. Par ailleurs, le passage délimité par le tube doit in situ toujours pouvoir rester ouvert et notamment à son extrémité proximale.

Compte-tenu des considérations ci-dessus, on réalisera le tube 1 de préférence en un matériau de dureté Shore situé entre 10 et 50, une silicone ou un latex pouvant convenir.

La paroi 8 du tube 1 est d'épaisseur variable selon le matériau utilisé, d'environ 2 mm par exemple, mais l'épaisseur peut varier en fonction de la dureté exigée.

Un matériau particulièrement appropriée est un silicone souple, polymérisable à température ambiante, et à contact alimentaire tel que le produit Silbione RTV 71557 (®) de Rhône-Poulenc". Des matériaux souples autres que les silicones ou les latex peuvent être utilisés tant qu'ils ont la dureté spécifiée et sont non irritants pour la peau et les muqueuses et dépourvus d'effet toxique pour l'organisme.

Le tube 1 peut être fait par dépôt du silicone sur un moule préformé, mais d'autres méthodes de réalisation sont possibles, en particulier par injection.

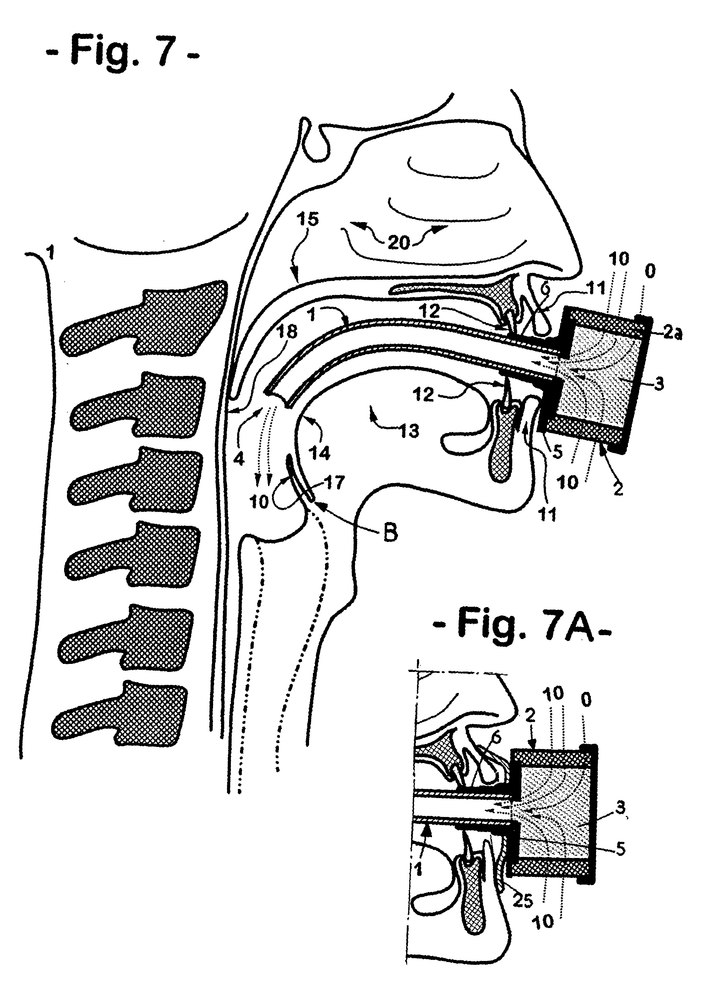

Grâce au tube souple 1, l'air inspiré 10 va donc librement de l'extérieur jusqu'au larynx, en passant l'obstacle de la zone B (figure 7) que constituent la langue 13, le voile du palais 15 et la base de langue 14. Il n'y a donc plus de gêne au passage de l'air, ainsi les ronflements sont supprimés, de même que les apnées.

En effet, les études récentes et les résultats insatisfaisants de la chirurgie du voile du palais tendent à prouver que l'obstacle au flux aérien, responsable de la vibration des tissus mous ou de l'obstruction complète de la voie aérienne, est situé (figure 6) au niveau de la zone B où se rejoignent et se collabent le voile du palais 15, la luette 16, la base de langue 14, contre la paroi pharyngée postérieure 18, et les amygdales 23 qui viennent rétrécir le pharynx en largeur.

Grâce à l'invention, le dispositif permet le passage de l'air, en évitant les obstacles anatomiques de la zone B qui se collabent lors du sommeil. Le flux aérien 10 va donc, sans frein et sans bruit, jusqu'au larynx.

Selon une autre caractéristique de l'invention, une base extra-buccale 5 vient appuyer contre les dents 12 et les lèvres 11. En outre, les premiers centimètres distaux du tube 1 sont renforcés par une partie en forme de douille ou renfort 6 réalisé en un matériau autre que celui du tube 1 afin d'en augmenter la rigidité pour l'appui des dents 12. Les dents viennent s'appuyer sur ce renfort 6 qui s'évase vers l'extrémité distale, et la base 5 extra-buccale va, elle, s'appuyer sur les lèvres 11. A titre d'exemple, la base 5 et le renfort 6 peuvent être réalisés en ABS (Acrylonitrile-Butadiène-Styrène), en polystyrène ou en un autre matériau analogue. Un métal tel que l'aluminium ou le titane peut également être utilisé.

Selon une autre caractéristique importante de l'invention, un filtre 2 de surface filtrante suffisante est placé à l'extrémité distale du tube 1, afin de filtrer et d'humidifier l'air inhalé par une partie filtrante 3 en un matériau adapté. Ce filtre est solidaire de la base 5 par tout moyen approprié. En effet, il est indispensable de remplacer la fonction nasale en débarrassant l'air des particules en suspension, en l'humidifiant, et en le réchauffant. En l'absence d'un tel filtre, l'air dessécherait rapidement la muqueuse laryngée et trachéale et le dispositif ne serait pas toléré. Les poussières inhalées pendant le sommeil seraient également un facteur d'intolérance du dispositif en provoquant irritations et toux.

Ce filtre peut avoir diverses formes et être constitué de matériaux variés. Dans le présent mode de réalisation, il comporte une cage annulaire 2a perméable à l'air dans laquelle est placée la partie filtrante 3 qui peut être en une mousse synthétique à mailles lâches, telle qu'une mousse en PVC (Poly-Chlorure de Vinyle), ou également en plaques de fibres agglomérées Polyamides ou Polyesters, mais d'autres matériaux sont utilisables, tant qu'ils ne freinent pas le passage de l'air 10. Ceci exclut une installation interne au tube 1 (ce qui est le cas dans le dispositif du brevet US précité). De préférence, la partie filtrante 3 est amovible pour pouvoir être changée ou nettoyée. La cage 2a, quant à elle peut être réalisée en ABS, comme la base 5 et le renfort 6.

On peut également prévoir un système de fixation du filtre 2 sur le tube 1 de telle sorte que l'on puisse changer la totalité du filtre, celui-ci étant alors fixe ou amovible.

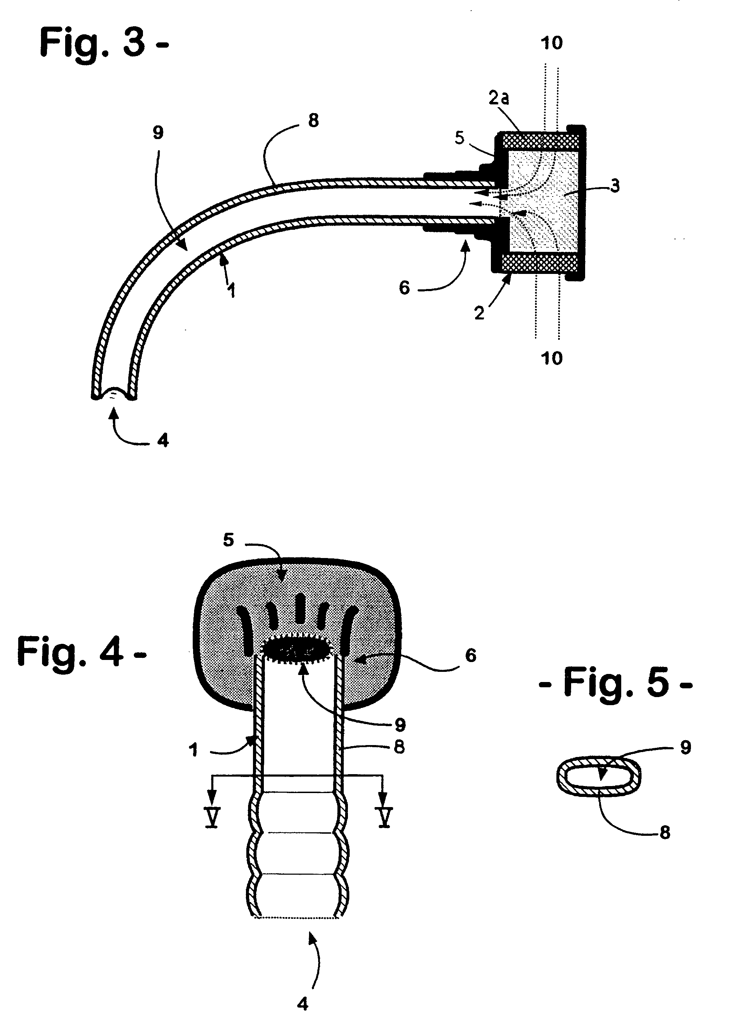

Dans le mode de réalisation représenté, le tube principal 1 a en coupe (figure 5) une forme ovalaire, de 20 mm de large sur 10 mm de haut environ, dans ses dimensions extérieures. La lumière interne 9 est de 16 mm en largeur et de 6 mm en hauteur environ. Ces dimensions peuvent être différentes selon la taille du tube et le matériau utilisé pour celui-ci. La section aplatie ou ovalaire permet d'offrir une lumière 9 et donc un débit d'air suffisant, sans qu'il y ait de frein au flux aérien 10, pour que celui-ci soit silencieux, et aussi pour que le dispositif soit bien supporté pendant le sommeil. Il est à noter que sur le schéma de la figure 7, les dimensions du tube 1 par rapport à l'anatomie de l'individu ne sont pas à l'échelle réelle pour permettre la mise en évidence du fonctionnement de l'appareil. On peut s'en apercevoir en comparant la figure 6 à la figure 7.

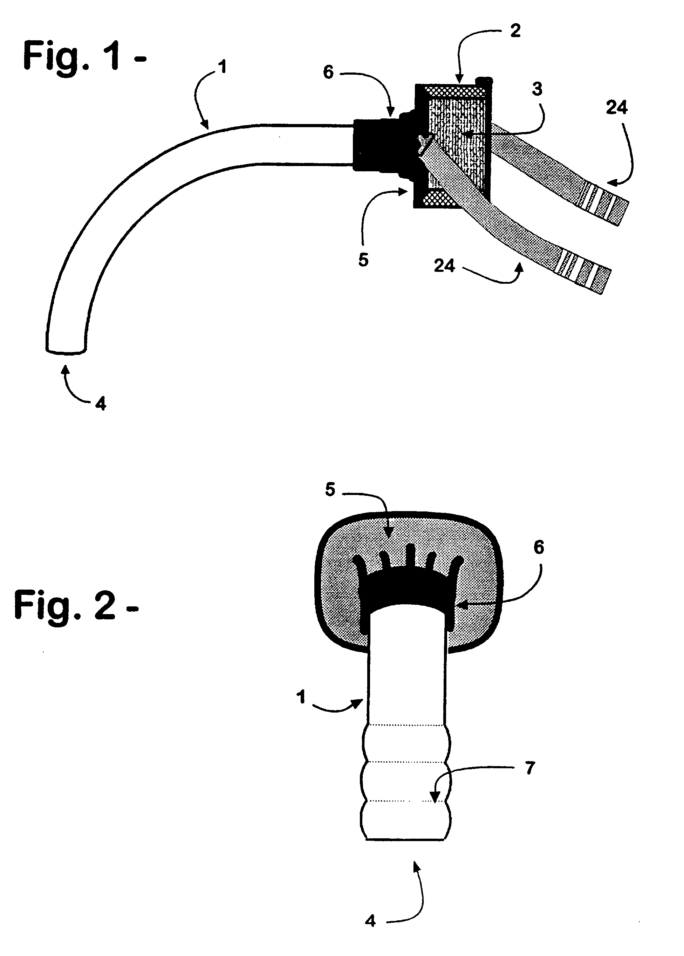

Selon l'anatomie de chacun, la longueur du tube 1 peut être adaptable pour chaque patient par simple section de l'extrémité proximale 4 du tube 1 afin que l'orifice proximal ne vienne buter contre l'épiglotte 17, ce qui provoquerait des douleurs ou un réflexe nauséeux ou de toux. Aussi, l'extrémité 4 du tube 1 doit pouvoir être facilement sectionnée avec de simples ciseaux. Pour faciliter cette section, dans le présent mode de réalisation, l'extrémité proximale 4 du tube 1 est pourvue de crans, saillies ou marques particulières 7 (figure 2) pour préciser et faciliter la section, afin que la longueur puisse progressivement être adaptée.

La paroi 8 du tube 1, la base 5 et le renfort 6 du tube 1 peuvent être armés d'un autre matériau, métallique ou synthétique afin d'en augmenter la rigidité, tel que par exemple des fils, des plaques ou d'autres matériaux. Il peut s'agir d'un renforcement interne, dans la paroi 8, la base et/ou le renfort 6 eux-mêmes, ou à l'extérieur de ceux-ci.

Un système de fixation est souhaitable afin de faire tenir le dispositif dans l'oro-pharynx pendant le sommeil. Le système le plus simple peut être constitué par un ou plusieurs élastiques 24 passant derrière les oreilles, la tête ou le cou.

La figure 7A illustre une variante du dispositif selon l'invention, dans laquelle il est prévu un capuchon 25 enfilé sur le renfort 6 contre la base 5. Ce capuchon, en un matériau souple, par exemple le même que celui du tube, présente à l'état libre une forme à peu près conique avec sa convexité tournée vers l'extrémité distale du tube 1. Ce capuchon 25 vient s'appuyer contre l'extérieur des lèvres en se déformant lorsque le dispositif est mis en place, créeant ainsi un moyen d'étanchéité évitant l'écoulement de salive vers l'extérieur à l'interface entre la base 5 et la sortie de la cavité buccale.

## Revendications

1. Dispositif de traitement de la ronchopathie et des apnées du sommeil, comprenant :
un tube (1) de forme anatomique courbée et destiné, lorsqu'il est en place dans l'oro-pharynx d'un patient à s'étendre par une extrémité proximale à partir d'au moins la base de la langue (14) jusqu'à faire saillie de la cavité buccale par une extrémité distale,
une base extra-buccale (5) solidaire dudit tube (1) et destinée, au cours de l'utilisation du dispositif, à venir s'appuyer contre les lèvres et les dents du patient, et
un filtre (2) également solidaire dudit tube (1) et placé à l'extrémité distale de celui-ci pour filtrer l'air passant dans le tube (1) vers l'intérieur de l'oro-pharynx du patient, et des moyens de renfort (5) associés audit tube pour éviter l'écrasement de celui-ci dans une zone prédéterminée de sa longueur au cours de son utilisation,
**caractérisé en ce que**
lesdits moyens de renfort (6) sont disposés sur ledit tube du côté de ladite base extra-buccale (5) tourné vers l'extrémité proximale du tube pour renforcer ledit tube dans la zone des dents et des lèvres.

2. Dispositif selon la revendication 1, **caractérisé en ce que** la base extra-buccale (5) est solidaire desdits moyens de renfort (6).

3. Dispositif selon la revendication 2 **caractérisé en ce que** la base (5) et/ou lesdits moyens de renfort (6) sont constitués d'un matériau d'une dureté supérieure à celle du matériau du tube (1).

4. Dispositif selon l'une des revendications 1 à 3, **caractérisé en ce que** le filtre (2) est fixe ou amovible.

5. Dispositif selon l'une quelconque des revendications précédentes, **caractérisé en ce que** la section du tube (1) est aplatie.

6. Dispositif selon l'une quelconque des revendications 1 à 5, **caractérisé en ce que** la longueur du tube (1) est adaptable pour chaque patient par simple section de son extrémité proximale (4).

7. Dispositif selon la revendication 6, **caractérisé en ce que** l'extrémité proximale du tube (1) est pourvue de crans, saillies ou marques particulières (7) pour préciser et faciliter la section.

8. Dispositif selon l'une quelconque des revendications 1 à 7, **caractérisé en ce que** le tube (1) est fait d'un matériau souple, non rigide et non irritant pour la peau et les muqueuses, tout en étant non toxique pour l'organisme, sa dureté Shore se situant entre 10 et 50.

9. Dispositif selon la revendication 8, **caractérisé en ce que** le matériau dudit tube (1) est une silicone ou un latex.

10. Dispositif selon l'une quelconque des revendications 1 à 9, **caractérisé en ce que** la paroi (8) du tube (1) est armée d'un autre matériau afin d'en modifier la rigidité.

11. Dispositif selon l'une quelconque des revendications 1 à 10 **caractérisé en ce qu'**il comporte un système de fixation comprenant des liens élastiques (24) attachés à ladite base (5) et destinés à être passés derrière les oreilles, la tête ou le cou, afin de faire tenir le dispositif dans l'oro-pharynx pendant le sommeil.

12. Dispositif selon l'une quelconque des revendications précédentes, **caractérisé en ce qu'**un capuchon (25) est positionné sur ledit tube (1) autour du renfort pour former un moyen d'étanchéité.

## Patentansprüche

1. Vorrichtung zur Behandlung des Schnarchens und des Atemstillstandes im Schlaf mit
einem Rohr (1) in anatomisch gebogener Form und das dazu bestimmt ist, wenn es im Mund-Rachenraum eines Patienten angeordnet ist, sich mit einem proximalen Ende ausgehend von zumindest der Basis der Zunge (14) zu erstrecken, bis es aus dem Mundraum mit einem distalen Ende vorspringt,
einer extra-bukkalen Basis (5), die mit dem Rohr (1) fest verbunden und dazu bestimmt ist, sich bei Benutzung der Vorrichtung an den Lippen und den Zähnen des Patienten abzustützen, und
einem Filter (2), das ebenfalls fest mit dem Rohr (1) verbunden und am distalen Ende von diesem angeordnet ist, um die Luft zu filtern, die durch das Rohr (1) zum Inneren des Mund-Rachenraumes des Patienten hindurchgeht, und Verstärkungsmitteln (6), die dem Rohr zugeordnet sind, um sein Zusammendrücken in einem vorbestimmten Bereich seiner Länge während seiner Benutzung zu vermeiden,
**dadurch gekennzeichnet, daß**
die Verstärkungsmittel (6) auf dem Rohr auf der Seite der außerhalb des Mundes angeordneten Basis (5) zum proximalen Ende des Rohres gerichtet angeordnet sind, um das Rohr im Bereich der Zähne und der Lippen zu verstärken.

2. Vorrichtung gemäß Anspruch 1, **dadurch gekennzeichnet, daß** die extra-bukkale Basis (5) fest mit den Verstärkungsmitteln (6) verbunden ist.

3. Vorrichtung gemäß Anspruch 2, **dadurch gekennzeichnet, daß** die Basis (5) und/oder die Verstärkungsmittel (6) aus einem Material gebildet sind, das eine Festigkeit hat, die derjenigen des Materials des Rohres (1) überlegen ist.

4. Vorrichtung gemäß einem der Ansprüche 1 bis 3, **dadurch gekennzeichnet, daß** das Filter (2) fest oder lösbar ist.

5. Vorrichtung gemäß irgendeinem der vorstehenden Ansprüche, **dadurch gekennzeichnet, daß** der Querschnitt des Rohres (1) abgeflacht ist.

6. Vorrichtung gemäß irgendeinem der Ansprüche 1 bis 5, **dadurch gekennzeichnet, daß** die Länge des Rohres (1) an jeden Patienten durch einfaches Abschneiden seines proximalen Endes (4) anpaßbar ist.

7. Vorrichtung gemäß Anspruch 6, **dadurch gekennzeichnet, daß** das proximale Ende des Rohres (1) mit Einkerbungen, Vorsprüngen oder speziellen Markierungen (7) versehen ist, um das Abschneiden zu präzisieren und zu erleichtern.

8. Vorrichtung gemäß irgendeinem der Ansprüche 1 bis 7, **dadurch gekennzeichnet, daß** das Rohr (1) aus einem flexiblen, nicht starren und nicht die Haut und die Schleimhaut reizenden Material hergestellt ist, wobei es nicht toxisch für den Organismus ist und seine Shorehärte zwischen 10 und 50 liegt.

9. Vorrichtung gemäß Anspruch 8, **dadurch gekennzeichnet, daß** das Material des Rohres (1) ein Silikon oder ein Latex ist.

10. Vorrichtung gemäß irgendeinem der Ansprüche 1 bis 9, **dadurch gekennzeichnet, daß** die Wand (8) des Rohres (1) mit einem anderen Material bewehrt ist, um seine Steifheit zu verändern.

11. Vorrichtung gemäß irgendeinem der Ansprüche 1 bis 10, **dadurch gekennzeichnet, daß** sie ein Befestigungssystem aufweist, das elastische Bänder (24) umfaßt, die an der Basis (5) befestigt und dazu bestimmt sind, hinter den Ohren, dem Kopf oder dem Hals entlanggeführt zu werden, um die Vorrichtung im Mund-Rachenraum während des Schlafes zu halten.

12. Vorrichtung gemäß irgendeinem der vorstehenden Ansprüche, **dadurch gekennzeichnet, daß** eine Kappe (25) auf dem Rohr (1) um die Verstärkung angeordnet ist, um ein Mittel zur Abdichtung zu bilden.

## Claims

1. Device for treating ronchopathy and sleep apnoea adapted to be placed in the oro-pharynx and in the pre-buccal region, **characterized in that** it comprises in combination:
an anatomical shape curved tube (1) so adapted that, when it is in situ in the oro-pharynx of a patient, it extends with a proximal end from at least the base (14) of the tongue until projecting with its distal end out of the buccal cavity,
an extra-buccal base (5) rigid with said tube (6) and adapted, when the device is in use, to bear against a patient's teeth and the lips (11), and
a filter (2) also rigid with said tube (1) and located at the distal end thereof in order to filter the air flowing through said tube (1) towards the patient's oro-pharynx, and reinforcing means (6) associated with said tube so as to prevent, in use, crushing thereof in a predetermined zone of its length,
**characterised in that**
said reinforcing means (6) are provided on said tube at the side of an extra-buccal base (5) oriented towards the proximal end of the tube so as to reinforce said tube in the region of the teeth and the lips.

2. Device according to Claim 1, **characterised in that** the extra-buccal base (5) is rigid with said reinforcing means (6).

3. Device according to Claim 2, **characterised in that** the base (5) and/or said reinforcing means (6) are made of a material having a hardness greater than that of the material of the tube (1).

4. Device according to any one of Claim 1-3, **characterised in that** the filter (2) is fixed or removable.

5. Device according to any one of Claims 1-4, **characterised in that** the section of the tube (1) is flattened.

6. Device according to anyone of claims 1-5, **characterised in that** the length of the tube (1) can be adapted to suit each patient by simply cutting off its proximal end (4).

7. Device according to Claim 6 **characterised in that** the proximal end of the tube (1) has notches, projections or special marks (7) to specify and facilitate cutting.

8. Device according to any one of Claims 1-7, **characterised in that** the tube (1) is made of a flexible, non-rigid material that is non-irritant for the skin and the mucous membranes and non-toxic to the organism, its Shore hardness being in the range 10 to 50.

9. Device according to Claim 8, **characterised in that** the material of said tube (1) is silicone or latex.

10. Device according to any one of Claims 1-9, **characterised in that** the wall (8) of the tube (1) is reinforced with another material to modify its rigidity.

11. Device according to any one of Claims 1-10, **characterised in that** it includes a fixing system comprising elastic bands (24) attached to said base (5) and adapted to be passed behind the ears, the head or the neck in order to hold the device in the oro-pharynx during sleep.

12. Device according to any one of the preceding Claims, **characterised in that** a cap (25) is positioned on said tube (1) around the reinforcement to form a seal.
